# FASCICULE DE BREVET EUROPEEN

(11) **EP 0 573 554 B1**
(45) Date de publication et mention de la délivrance du brevet: **27.09.1995**
(21) Numéro de dépôt: 92907314.6
(22) Date de dépôt: 02.03.1992
(51) Int. Cl.: A61K 7/48, A61K 38/01, C07K 7/06

(54) **UTILISATION COMME INGREDIENT ACTIF, DANS LA PREPARATION D'UNE COMPOSITION COSMETIQUE, D'AU MOINS UN PEPTIDE PURIFIE POUVANT ETRE OBTENU PAR HYDROLYSE DE CASEINE A ACTIVITE OPIOIDE**
VERWENDUNG VON MINDESTENS EINEM GEREINIGTEN PEPTID, WELCHES DURCH KASEINHYDROLYSE HERGESTELLT WERDEN KANN UND OPIATAKTIVITÄT HAT, ALS AKTIVEN BESTANDTEIL IN DER HERSTELLUNG EINER KOSMETISCHEN ZUSAMMENSETZUNG
USE OF AT LEAST ONE PURIFIED PEPTIDE OBTAINABLE BY HYDROLYSIS OF CASEIN AND HAVING OPIOID ACTIVITY AS ACTIVE PRINCIPLE IN THE MANUFACTURE OF A COSMETIC COMPOSITION

(30) Priorité: 01.03.1991 FR 9102510
(43) Date de publication de la demande: 15.12.1993
(73) Titulaire: L'OREAL, 75008 Paris (FR)
(72) Inventeur: BITRI, Lofti, F-34000 Montpellier (FR); BAREY, Michel, F-92330 Sceaux (FR); DARMANTON, Patrick, F-92320 Bourg-la-Reine (FR)
(74) Mandataire: Tonnellier, Jean-Claude
(86) Numéro de dépôt international: FR9200191
(87) Numéro de publication internationale: WO9215279

(56) Documents cités:
- EP-A- 0 019 829
- US-A- 3 061 512
- US-A- 3 558 770
- US-A- 4 524 136
- CHEMICAL ABSTRACTS, vol. 97, no. 21, 22 Novembre 1982, Columbus, Ohio, US; abstract no. 175260Q, C. ZIOUDROU ET AL: 'Synthetic opioid alpha-casein peptides. Structure activity relationship'
- CHEMICAL ABSTRACTS, vol. 99, no. 15, 10 Octobre 1983, Columbus, Ohio, US; abstract no. 118021E, S. LOUKAS ET AL.: 'Opioid activities and structures of alpha-casein-derived exorphins'
- CHEMICAL ABSTRACTS, vol. 98, no. 7, 14 Février 1983, Columbus, Ohio, US; abstract no. 52128R, V. BRANTL ET AL.: 'Opiate-like acting compounds in milk and milk products. 2. Isolation, structure analysis and biological effects of beta-casomorphins'
- CHEMICAL ABSTRACTS, vol. 92, no. 3, 21 Janvier 1980, Columbus, Ohio, US; abstract no. 15233B, AGNES HENSCHEN ET AL.: 'Novel opioid peptides derived from casein (beta-casomorphins). II. Structure of active components from bovine casein peptone'

## Description

La présente invention a pour objet l'utilisation comme ingrédient actif, dans la préparation d'une composition cosmétique, d'au moins un peptide pouvant être obtenu par hydrolyse ou protéolyse de caséines du lait.

On sait que les caséines sont des protéines phosphorées qui précipitent à partir du lait écrémé, à un pH de 4,6 à température ambiante.

On connaît, parmi les constituants des caséines, quatre constituants majeurs : les caséines alphaₛ₁, alphaₛ₂, bêta et Kappa.

On sait que certains peptides pouvant être obtenus par hydrolyse de caséine ont une activité de type opioïde, cette activité pouvant être mise en évidence notamment par l'effet inhibiteur de la contraction de l'iléon de cobaye stimulé électriquement. Cette inhibition est réversible sous l'action de la naloxone, qui est un antagoniste spécifique de la morphine. L'activité des peptides de caséine peut également être mise en évidence par l'inhibition des contractions induites par stimulation électrique du vas deferens de la souris ou du rat, cette inhibition étant ici encore réversible sous l'action de la naloxone.

Le document EP-A-19829 décrit des peptides dérivés de caséine (bêta-casomorphines), utilisables comme médicaments ayant une activité opioïde et neuroleptique.

Le document US-3558770 décrit notamment l'utilisation d'hydrolysats totaux de caséine ayant un effet de camouflage des rides de la peau.

On a maintenant découvert que les peptides à activité opioïde pouvant être obtenus notamment par hydrolyse ou protéolyse de caséines du lait, ou des fractions peptidiques les contenant, ont des propriétés cosmétiques intéressantes, qui permettent de les utiliser comme ingrédients actifs dans des compositions cosmétiques.

La composition cosmétique obtenue selon l'invention est caracterisée par le fait qu'elle contient, comme ingrédient actif, au moins un peptide à activité opioïde pouvant être obtenu par hydrolyse ou protéolyse de caséines du lait. La composition de l'invention est notamment une composition cosmétique pour la peau.

L'ingrédient actif peut être au moins un peptide purifié à activité opioïde, obtenu soit par synthèse peptidique, soit par purification d'un hydrolysat de caséïnes. L'ingrédient actif peut également être constitué par une fraction peptidique contenant au moins un peptide à activité opioïde, cette fraction pouvant être obtenue par purification partielle d'un hydrolysat total de caséïnes, par exemple par les méthodes chromatographiques, par exemple par chromatographie d'adsorption, chromatographie d'exclusion (tamisage moléculaire) ou chromatographie d'affinité, ou par les méthodes de filtration sur membranes, notamment par ultrafiltration.

Les peptides ou fractions peptidiques utilisés comme ingrédients actifs dans les compositions de l'invention sont donc au moins partiellement purifiés, ce qui exclut l'incorporation comme ingrédient actif d'un hydrolysat total de caséine.

Les peptides présents comme ingrédients actifs dans les compositions cosmétiques obtenues selon l'invention sont choisis parmi :
- l'heptapeptide :
   Arg Tyr Leu Gly Tyr Leu Glu
   ou l'hexapeptide correspondant :
   Arg Tyr Leu Gly Tyr Leu.
Les deux peptides ci-dessus ont été isolés à partir d'hydrolysats d'alphaₛ₁ caséine ; voir par exemple C. ZIOUDROU et al, J.BIOL CHEM 254, n°7, 2446-2449 (1979).
- L'undécapeptide :
   Tyr Pro Phe Pro Gly Pro Ile Pro Asn Ser Leu,
- L' heptapeptide correspondant :
   Tyr Pro Phe Pro Gly Pro Ile (encore appelé bêta-casomorphine-7),
- et le pentapeptide correspondant :
   Tyr Pro Phe Pro Gly (encore appelé bêta-casomorphine-5).

On sait que l'undécapeptide, à activité opioïde modérée, lui-même précurseur de l'hepta et du pentapeptide à activité plus importante, provient de la bêta-caséine ; voir par exemples BRANTL et al., Hoppe-Seyler's Z. Physiol. Chem. 360, 1211-1216 (1979) et Life Sci. 28, 1903-1909 (1981).

Certains peptides à activité opioïde qui sont mentionnés ci-dessus peuvent également être préparés par hydrolyse ou protéolyse de gluten de blé ou de protéines de soja ; voir par exemple C. ZIOUDROU et al., article déjà cité.

En outre, la synthèse de certains de ces peptides a été décrite par Spyros Loukas et al., Biochemistry 22 (19), pp 4567-4573 (1983).

Généralement, le ou les peptides à activité opioïde sont présents, dans la composition obtenue selon l'invention, à raison de 0,1 à 5 % en poids.

On a en outre découvert un procédé particulièrement intéressant pour préparer des peptides ou fractions peptidiques utilisables dans lesdites compositions cosmétiques.

Ce procédé est caractérisé par le fait que :
a) soit l'on soumet une solution de caséine à une hydrolyse acide à un pH non supérieur à 4,0 et à une température de 20 à 100°C,
b) soit l'on soumet une solution de caséine à l'action d'une protéase de Staphylococcus aureus,
   puis que, dans les deux cas, on purifie selon les méthodes usuelles les fractions peptidiques obtenues et réunit les fractions peptidiques purifiées ayant une activité opioïde.

Dans des modes de réalisation particuliers, le procédé peut également présenter les caractéristiques suivantes, prises isolément ou en combinaison :
- on effectue par exemple l'hydrolyse acide à un pH de 3,5 environ ; la concentration de la caséine dans la solution de départ est par exemple de 1 à 10 % en poids ; la caséine de départ est par exemple une caséine alcalisoluble, ou une caséine selon Hammarsten, ou encore une caséine précipitée au pH isoélectrique à partir d'un lait écrémé ; la durée de l'hydrolyse dépend de la température ; on peut déterminer facilement dans chaque cas la durée de la réaction d'hydrolyse (généralement entre 15 min et 70 heures environ), de préférence en déterminant par des expériences de routine le temps d'apparition d'une activité opioïde optimale dans les fractions peptidiques obtenues ;
- on effectue la réaction de protéolyse en utilisant de 25 à 50 unités d'enzyme par mg de caséine de départ ; la caséine de départ est notamment une alpha-caséine ; le temps optimal de la réaction peut être déterminé par électrophorèse en gel de polyacrylamide 17,5 % (SDS-PAGE) : par dépôts successifs d'aliquotes du milieu réactionnel enrichi en SDS (0,5 %) et en glycérol (10 %). L'hydrolyse s'accompagne d'une disparition progressive de la bande correspondant à l'alpha-caséine et de l'apparition de fractions protéiques de poids moléculaire inférieur.

La protéase de S. aureus est par example celle de S. aureus Strain V8 type XVII-B commercialisée par SIGMA ;
- selon une variante, on peut faire précéder la protéolyse enzymatique d'un clivage de la caséine de départ avec du bromure de cyanogène, dont on sait qu'il coupe les liaisons peptidiques dans lesquelles une méthionine est impliquée par son carboxyle ; on utilise le bromure de cyanogène généralement à raison de 2,5 g/g d'alpha_{S}-caséine ; on opère par exemple en solution d'acide formique a 70 % ; voir OTANI et al. AGR.BIOL.CHEM. 50(3)-607-1986 ;
- la purification des fractions peptidiques obtenues peut être faite selon les méthodes usuelles, notamment par chromatographie d'exclusion ou par échanges d'ions ou par interactions hydrophobes ; après mise au point du procédé de purification par des expériences de routine, on pourra opérer par exemple de façon à isoler et/ou à réunir les fractions peptidiques ayant une activté opioïde ; cette activité opioïde peut être déterminée selon les méthodes classiques telles que l'inhibition des contractions induites par stimulation électrique sur l'iléon de cobaye ou sur le vas deferens chez le rat ou la souris, cette inhibition étant levée par la présence d'un antagoniste de la morphine tel que la naloxone ; pour la description de ces tests classiques voir par exemple : Method used for the study of opioids receptors-LESLIE-Pharmacological Reviews-Vol.39, n° 3-1987.

On peut également purifier partiellement les fractions peptidiques à l'aide de membranes ayant un seuil de coupure approprié (par exemple 5000) qui laissent passer les peptides de masse moléculaire inférieure au seuil de coupure, et qui retiennent les protéines ou fragments protéiques de masse moléculaire supérieure, y compris les protéases utilisées dans le procédé de protéolyse.

Les fractions peptidiques utilisables comme ingrédient actif dans les compositions obtenues selon l'invention sont notamment les fractions exemptes de peptides de masse moléculaire supérieure à 5000, et en particulier de peptides de masse moléculaire supérieure à 3000.

Les peptides présents dans lesdites compositions peuvent également être obtenus selon des procédés analogues à partir de protéines du soja ou du gluten.

Les compositions de l'invention présentent notamment, lorsqu'elles sont appliquées sur la peau, un effet calmant et/ou régénérant, permettant d'en améliorer l'aspect.

Ces propriétés peuvent être utilisées notamment pour réaliser des compositions destinées à atténuer ou à éliminer les effets de l'irritation de la peau provoquée notamment par l'action du rayonnement solaire ou par l'action d'autres agents irritants physiques, chimiques ou biologiques ; ou encore des compositions permettant de lutter contre les effets du vieillissement de la peau, afin d'en améliorer l'aspect.

Les compositions obtenues selon l'invention contiennent les fractions peptidiques décrites ci-dessus en association avec un véhicule cosmétique approprié, c'est-à-dire un véhicule compatible avec l'application sur la peau et les muqueuses. Ledit véhicule cosmétique est un véhicule classique permettant de présenter la composition notamment sous la forme de solutions aqueuses ou hydroalcooliques, d'émulsions (laits ou crèmes), sous la forme de gels aqueux, sous la forme de solides comme les bâtons (colorés ou non) pour les lèvres, ou encore sous la forme d'aérosols.

Les excipients permettant de préparer ces diverses formes de compositions cosmétiques sont connus et décrits notamment dans les ouvrages classiques de cosmétologie.

Les compositions obtenues selon l'invention peuvent contenir en outre des adjuvants usuels tels que des parfums, des agents colorants, des agents épaississants, des agents séquestrants, des modificateurs de pH, des agents conservateurs, des filtres UV, des tensioactifs ou émulsifiants, des corps gras, des polymères, etc...

Ces compositions peuvent se présenter notamment sous la forme de gels, de laits, de crèmes, d'émulsions ou de pâtes constituant des compositions pour la toilette ou pour les soins de la peau, notamment après l'exposition au soleil, des compositions pour le bain, des compositions de protection contre le rayonnement solaire, des crèmes ou lotions pour les soins des mains, ou des bâtons pour les lèvres.

Ces compositions cosmétiques sont préparées selon les méthodes usuelles.

L'invention concerne en outre un procédé de traitement cosmétique consistant à appliquer sur la peau une quantité efficace d'une composition cosmétique telle que définie précédemment.

On a également découvert que les peptides décrits ci-dessus présentent, chez l'homme, au contact de la peau et des muqueuses, des propriétés pharmacologiques intéressantes, notamment des propriétés cicatrisantes et apaisantes. Ces propriétés peuvent être mises à profit dans des compositions telles que des baumes gingivaux, des solutions constituant des bains de bouche, des comprimés sublinguaux, des pastilles à sucer, des pâtes à mâcher, ou des encore des films ou pansements imprégnés. Ces compositions, qui contiennent lesdits peptides généralement à raison de 0,05 à 5 % en poids, en combinaison avec des excipients et adjuvants classiques, sont préparées selon les méthodes usuelles. L'invention a donc également pour objet l'utilisation des peptides tels que définis ci-dessus, éventuellement sous la forme d'hydrolysats ou de fractions peptidiques d'hydrolysats au moins partiellement purifiés, comme principes actifs dans la préparation d'une composition cicatrisante et/ou apaisante pour la peau et les muqueuses. Les compositions buccales peuvent en outre servir notamment au traitement des aphtes. L'invention concerne également un procédé de traitement correspondant de la peau ou des muqueuses, dans lequel on applique sur la peau ou les muqueuses, une quantité efficace d'une composition telle qu'elle vient d'être définie.

Les exemples suivants illustrent l'invention.

### EXEMPLES DE PREPARATION DE FRACTIONS PEPTIDIQUES

### EXEMPLE 1 : Hydrolyse de l'alpha-caséine par la protéase de Staphylococcus aureus.

La caséine de départ est l'alpha-caséine commercialisée par Sigma, qui contient 85 % d'alpha ₛ₁-caséine.

On solubilise l'alpha-caséine (5 g) dans 100 ml d'une solution tampon Tris HCl 125 mM, pH 6,8, en présence de 0,5 % de dodécylsulfate de sodium et de 10 % de glycérol. On ajoute la protéase de S.aureus Strain V8 Type XVII-B (commercialisée par Sigma) à raison de 50 unités/mg de caséine.

On laisse incuber à 37°C. L'hydrolyse de l'alpha-caséine peut être suivie par électrophorèse en gel de polyacrylamide, comme il a été indiqué précédemment.

En procédant à une étude systématique, et en fixant le temps d'hydrolyse à 3 heures, il est apparu que la concentration d'enzyme efficace pour hydrolyser la caséine est de l'ordre de 25 à 50 unités/mg de caséine.

On rappelle qu'une unité de protéase est la quantité d'enzyme qui hydrolyse 1 µM d'alpha-phényl ester de l'acide N-t-butoxycarbonyl-L-glutamique par minute à pH 7,8 et à 37°C..

Par ailleurs, en fixant la concentration enzymatique à 50 unités/mg de caséine, le temps d'hydrolyse est compris entre 2 et 3 heures environ.

En variante, il est possible d'effectuer un traitement préliminaire de clivage de l'alpha-caséine par le bromure de cyanogène suivi d'une purification des peptides par tamisage moléculaire.

Par exemple, on solubilise 100 mg d'alpha-caséine (Sigma) dans une solution d'acide formique à 70 %. On ajoute, en deux fois, 250 mg de bromure de cyanogène. On laisse réagir pendant 24 heures sous agitation. Les peptides obtenus sont séparés par tamisage moléculaire sur une colonne de Sephadex G50, équilibrée avec un tampon TEMED HCl 20 mM, pH 5,5, en présence d'urée 4,5 M. Le pic d'exclusion contient notamment un mélange de peptides ayant 61 et 63 résidus d'acides aminés, dont le second contient l'heptapeptide d'alphaₛ₁-caséine. Les peptides obtenus sont alors soumis à l'hydrolyse sous l'action de la protéase de S.aureus, comme décrit précédemment.

### EXEMPLE 2 : Hydrolyse acide.

On utilise conme produit de départ une solution de caséine selon Hammarsten (Merck réf. 2242) ou encore la caséine alcalisoluble (Merck réf. 2241).

On rappelle que la caséine alcalisoluble ainsi que la caséine selon Hammarsten sont des caséines totales (alpha+bêta+kappa).

On solubilise la caséine dans un tampon acétate 50 mM, ph 3,5, la concentration de la caséine pouvant varier de 1 à 10 % en poids.

Une étude systématique a montré que l'on peut opérer par exemple à température ambiante (20°C) pendant 70 heures, à 35°C pendant 50 heures, à 43°C pendant 45 heures ou à 100°C pendant 15 min, en agitant pendant toute la durée de l'hydrolyse.

Après centrifugation dans les conditions suivantes (30000 g ou mieux 50000 g pendant 15 minutes à 4°C) on obtient une fraction peptidique dont on a vérifié qu'elle présente des propriétés de type opioïde dans les tests sur iléon de cobaye et sur vas deferens de souris ou de rats.

La fraction peptidique peut être concentrée par exemple par lyophilisation puis amenée a un volume déteminé par addition d'eau distillée ou d'une solution de NaCl à 0,9 % ou encore d'une solution tampon.

On peut également purifier davantage la fraction peptidique obtenue par une opération de filtration sur gel, par exemple sur Sephadex G75 équilibré et élué avec un tampon Tris, HCl 50mM, pH 8,8. Par mesure de la densité optique des éluats à 220 nm on obtient un profil d'élution avec deux pics principaux : le deuxième pic correspond à une fraction ayant une activité de type opioïde plus importante.

Le rendement en peptides à activité opioïde est environ dix fois supérieur au rendement obtenu par protéolyse à l'aide de pepsine selon la méthode décrite par C. ZIOUDROU et al., article cité précédemment.

Pour l'utilisation dans les compositions cosmétiques selon l'invention, les fractions peptidiques (qui sont obtenues, par voie chimique comme par voie enzymatique, à pH acide) sont neutralisées par une base appropriée telle que l'hydroxyde de sodium.

### EXEMPLES DE COMPOSITIONS

### EXEMPLE A : Gel après-soleil

| CONSTITUANTS | % en poids |
|---|---|
| Hydrolysat de caséine obtenu selon l'exemple 1 | 3,00 |
| Polymère carboxyvinylique | 0,30 |
| Propylène glycol | 20,00 |
| Acide 2-hydroxy 4-méthoxybenzophénone 5-sulfonique | 0,05 |
| Sel de potassium de l'acide éthylène diaminetétracétique | 0,02 |
| Triéthanolamine | 0,42 |
| FDC Blue 1 | 0,0002 |
| Huile de ricin hydrogénée oxyéthylénée à 60 moles d'oxyde d'éthylène (OE) | 0,5 |
| Parfum | 0,20 |
| Parahydroxybenzoate de méthyle | 0,2 |
| Alcool éthylique à 96 % | 6,5 |
| Eau q.s.p. | 100,00 |

On peut remplacer l'hydrolysat de caséine par celui de l'exemple 2.

Cette composition a été préparée de la façon suivante :

Dans un mélangeur, on prépare le gel en laissant gonfler sous agitation pendant deux heures le polymère carboxylique dans une phase aqueuse chauffée à 70°C renfermant le conservateur, le propylène glycol et le sel de potassium de l'acide éthylènediaminetétracétique.

Puis à 40°C, on rajoute sous agitation :
- l'acide 2-hydroxy-4-méthoxybenzophénone 5-sulfonique solubilisé dans l'eau avec la triéthanolamine,
- l'hydrolysat de caséine préalablement solubilisé dans l'eau additionnée de parahydroxybenzoate de méthyle.
- le colorant
- le parfum peptisé par l'huile de ricin hydrogénée dans une phase contenant l'alcool éthylique et de l'eau.

Ce gel appliqué sur la peau a un effet protecteur et apaisant.

### EXEMPLE B : Emulsion apaisante après-soleil

| CONSTITUANTS | % en poids |
|---|---|
| Hydrolysat de caséine obtenu selon l'exemple 1 * | 1 |
| Mélange d'alcool cétylstéarylique et cétylstéarylique oxyéthyléné (33 OE) | 3 |
| Mono/distéarate de glycérol | 1 |
| Huile de vaseline | 6,5 |
| Myristate d'isopropyle | 3 |
| Polydiméthyl siloxane | 0,5 |
| Alcool cétylique | 1,0 |
| Ditertiobutyl-4-hydroxytoluène | 0,03 |
| Conservateur | 0,3 |
| Parfum | 0,3 |
| Eau q.s.p. | 100,00 |

| | |
|---|---|
| * ou de l'exemple 2 | |

On a préparé cette émulsion de la façon suivante :
La phase grasse est émulsionnée sous agitation à 65°-70°C avec la phase aqueuse renfermant tous les constituants hydrosolubles, sauf l'hydrolysat de caséine et le parfum. L'hydrolysat de caséine est rajouté sous agitation lorsque la température de l'émulsion atteint 35°C. Enfin, on introduit le parfum.

Cette émulsion est appliquée sur la peau pour son effet apaisant après une longue exposition au soleil et en cas de "coup de soleil".

### EXEMPLE C : Crème pour les lèvres

| CONSTITUANTS | % en poids |
|---|---|
| Cire émulsifiante (Polywax NP) | 6,00 |
| Mélange d'huile minérale et d'alcool de lanoline | 3,50 |
| Myristate de myristyle | 2,00 |
| Huile de silicone | 2,00 |
| Stéarate de glycérol | 1,20 |
| Cire d'abeilles | 0,80 |
| Cire de paraffine | 1,00 |
| Glycérol | 6,00 |
| Polyvinylpyrrolidone (PVP-K30 de GAF) | 2,00 |
| Hydrolysat de caséine obtenu à l'exemple 1 | 2,50 |
| Conservateur | 0,20 |
| Ammoniaque q.s.p. pH = 6,5 | |
| Eau | q.s.p. 100,00 |
| * ou à l'exemple 2 | |

Pour préparer cette crème, la phase grasse est maintenue à 70°C puis est émulsionnée dans la phase aqueuse chauffée à la même température. L'hydrolysat de caséine est ensuite ajouté à 30-35°C sous agitation, ainsi que (éventuellement) un parfum. Le pH est ajusté à froid.

Cette crème est particulièrement adaptée aux lèvres gercées ou crevassées, en particulier en, raison du froid.

### EXEMPLE D : BAUME GINGIVAL POUR BEBE

| CONSTITUANTS | % en poids |
|---|---|
| Hydrolysat de caséine obtenu selon l'exemple 1 | 2,00 |
| Propylène glycol | 5,00 |
| Glycérol | 20,00 |
| Acide polyacrylique | 2,00 |
| Agent aromatisant | 0,10 |
| Conservateurs | 0,15 |
| Hydroxyde de sodium, qsp pH = 7 | |
| Eau | q.s.p. 100,00 |

On peut remplacer l'hydrolysat de caséine par celui obtenu à l'exemple 2.

### Préparation

On réalise un gel en mélangeant l'acide polyacrylique, la moitié de l'eau et une partie du conservateur.

Le lendemain, on rajoute dans l'ordre et sous agitation :
- l'hydrolysat de caséine préalablement mélangé au glycérol,
- le reste du conservateur solubilise, dans l'eau
- l'agent aromatisant en solution à 2 % dans le propylène glycol
- l'hydroxyde de sodium en solution aqueuse
Une agitation de 45 minutes, avec dégazage, termine la fabrication.
- On applique ce baume sur les gencives des nouveau-nés pour calmer les douleurs des premières dents.

## Revendications

1. Utilisation comme ingrédient actif, dans la préparation d'une composition cosmétique, d'au moins un peptide, au moins partiellement purifié, choisi parmi :
Arg Tyr Leu Gly Tyr Leu Glu,
Arg Tyr Leu Gly Tyr Leu,
Tyr Pro Phe Pro Gly Pro Ile Pro Asn Ser Leu,
Tyr Pro Phe Pro Gly Pro Ile, et
Tyr Pro Phe Pro Gly.

2. Utilisation selon la revendication 1, dans la préparation d'une composition cosmétique destinée à améliorer l'aspect de la peau irritée par l'action du rayonnement solaire ou d'autres agents irritants, ou destinée à lutter contre les effets du vieillissement de la peau.

3. Utilisation selon la revendication 1 ou 2, caractérisée par le fait que ledit peptide est présent à raison de 0,1 à 10 % en poids, en particulier de 0,1 à 5 % en poids, dans ladite composition.

4. Utilisation selon l'une quelconque des revendications précédentes, caractérisée par le fait que ledit ingrédient actif est une fraction peptidique au moins partiellement purifiée, contenant au moins un peptide tel que défini dans la revendication 1.

5. Utilisation selon la revendication précédente, caractérisée par le fait que ladite fraction peptidique est exempte de peptides de masse moléculaire supérieure à 5000.

6. Utilisation selon la revendication précédente, caractérisée par le fait que ladite fraction peptidique est exempte de peptides de masse moléculaire supérieure à 3000.

7. Utilisation selon l'une quelconque des revendications précédentes, caractérisée par le fait que ledit peptide ou ladite fraction peptidique peut être obtenu par un procédé dans lequel :
a) soit l'on soumet une caséine à une hydrolyse acide à un pH non supérieur à 4,0 et à une température de 20 à 100°C,
b) soit l'on soumet une caséine à l'action d'une protéase de Staphylococcus aureus,
puis, dans les deux cas, on purifie selon les méthodes usuelles les fractions peptidiques obtenues.

8. Utilisation selon l'une quelconque des revendications précédentes, caractérisée par le fait que l'on mélange ledit ingrédient actif avec un véhicule cosmétique approprié.

9. Utilisation selon l'une quelconque des revendications précédentes, caractérisée par le fait que ladite composition se présente sous la forme d'une solution aqueuse ou hydroalcoolique, d'une émulsion, d'un gel aqueux, ou sous la forme d'un bâton, coloré ou non, pour les lèvres.

10. Procédé de traitement cosmétique, caractérisé par le fait que l'on applique sur la peau une quantité efficace d'une composition cosmétique préparée comme défini dans l'une quelconque des revendications 1 à 9.

11. Procédé selon la revendication précédente, caractérisé par le fait que l'on applique ladite composition pour améliorer l'aspect de la peau irritée par l'action du rayonnement solaire ou d'autres agents irritants, ou pour lutter contre les effets du vieillissement de la peau.

## Claims

1. Use as active ingredient, in the preparation of a cosmetic composition, of at least one peptide, which is at least partially purified, chosen from:
Arg Tyr Leu Gly Tyr Leu Glu,
Arg Tyr Leu Gly Tyr Leu,
Tyr Pro Phe Pro Gly Pro Ile Pro Asn Ser Leu,
Tyr Pro Phe Pro Gly Pro Ile, and
Tyr Pro Phe Pro Gly.

2. Use according to Claim 1, in the preparation of a cosmetic composition intended to improve the appearance of skin irritated by the action of solar radiation or other irritants, or intended to combat the effects of ageing of the skin.

3. Use according to Claim 1 or 2, characterized in that the said peptide is present in an amount of from 0.1 to 10% by weight, in particular from 0.1 to 5% by weight, in the said composition.

4. Use according to any one of the preceding claims, characterized in that the said active ingredient is an at least partially purified peptide fraction containing at least one peptide as defined in Claim 1.

5. Use according to the preceding claim, characterized in that the said peptide fraction is free of peptides with a molecular weight greater than 5000.

6. Use according to the preceding claim, characterized in that the said peptide fraction is free of peptides with a molecular weight greater than 3000.

7. Use according to any one of the preceding claims, characterized in that the said peptide or the said peptide fraction may be obtained by a process in which:
a) either a casein is subjected to acidic hydrolysis at a pH not above 4.0 and at a temperature of from 20 to 100°C,
b) or a casein is subjected to the action of a Staphylococcus aureus protease,
and, in both cases, the peptide fractions obtained are then purified according to the usual methods.

8. Use according to any one of the preceding claims, characterized in that the said active ingredient is mixed with a suitable cosmetic vehicle.

9. Use according to any one of the preceding claims, characterized in that the said composition is in the form of an aqueous or aqueous-alcoholic solution, an emulsion or an aqueous gel, or in the form of a coloured or colourless stick for the lips.

10. Cosmetic treatment process, characterized in that an effective amount of a cosmetic composition prepared as defined in any one of Claims 1 to 9 is applied to the skin.

11. Process according to the preceding claim, characterized in that the said composition is applied in order to improve the appearance of skin irritated by the action of solar radiation or other irritants, or in order to combat the effects of ageing of the skin.

## Patentansprüche

1. Verwendung mindestens eines Peptids, das mindestens teilweise gereinigt und aus folgender Gruppe ausgewählt ist
Arg Tyr Leu Gly Tyr Leu Glu,
Arg Tyr Leu Gly Tyr Leu,
Tyr Pro Phe Pro Gly Pro Ile Pro Asn Ser Leu,
Tyr Pro Phe Pro Gly Pro Ile und
Tyr Pro Phe Pro Gly
als aktiver Bestandteil bei der Herstellung einer kosmetischen Zubereitung.

2. Verwendung nach Anspruch 1 bei der Herstellung einer kosmetischen Zubereitung zur Verbesserung des Aussehens einer durch Sonnenstrahlen oder andere irritierende Agenzien irritierten Haut oder zur Bekämpfung von Alterungserscheinungen der Haut.

3. Verwendung nach Anspruch 1 oder 2, dadurch gekennzeichnet, daß das Peptid in einer Menge von 0,1 bis 10 Gew.%, im besonderen 0,1 bis 5 Gew.% in der Zubereitung vorliegt.

4. Verwendung nach einem der vorangehenden Ansprüche, dadurch gekennzeichnet, daß der aktive Bestandteil eine mindestens teilweise gereinigte Peptid-Fraktion ist, die mindestens ein Peptid gemäß Anspruch 1 enthält.

5. Verwendung nach einem der vorangehenden Ansprüche, dadurch gekennzeichnet, daß die Peptid-Fraktion frei von Peptiden mit einem Molekulargewicht oberhalb von 5000 ist.

6. Verwendung nach einem der vorangehenden Ansprüche, dadurch gekennzeichnet, daß die Peptid-Fraktion frei von Peptiden mit einem Molekulargewicht oberhalb von 3000 ist.

7. Verwendung nach einem der vorangehenden Ansprüche, dadurch gekennzeichnet, daß das Peptid oder die Peptid-Fraktion durch ein Verfahren erhältlich ist, bei dem man
a) entweder ein Kasein einer Säurehydrolyse bei einem pH nicht über 4,0 bei einer Temperatur von 20 bis 100 °C unterwirft, oder
b) ein Kasein der Einwirkung einer Protease von Staphylococcus aureus unterwirft, und
in beiden Fällen die erhaltenen Peptid-Fraktionen nach bekannten Verfahren reinigt.

8. Verwendung nach einem der vorangehenden Ansprüche, dadurch gekennzeichnet, daß man den wirksamen Bestandteil mit einem geeigneten kosmetischen Trägerstoff vermischt.

9. Verwendung nach einem der vorangehenden Ansprüche, dadurch gekennzeichnet, daß die Zubereitung in Form einer wäßrigen oder hydroalkoholischen Lösung, einer Emulsion, eines wäßrigen Gels oder in Form eines gefärbten oder nicht gefärbten Lippenstiftes vorliegt.

10. Kosmetisches Behandlungsverfahren, dadurch gekennzeichnet, daß man auf die Haut eine wirksame Menge einer kosmetischen Zubereitung aufbringt, die nach einem der Verfahren 1 bis 9 hergestellt wurde.

11. Verfahren nach einem der vorangehenden Ansprüche, dadurch gekennzeichnet, daß man die Zubereitung zur Verbesserung des Aussehens der Haut, die durch Sonnenbestrahlung oder andere irritierende Agenzien geschädigt ist oder zur Bekämpfung von Alterungserscheinungen der Haut aufbringt.
